# EUROPEAN PATENT APPLICATION

(11) **EP 1 514 530 A2**
(43) Date of publication of application: **16.03.2005**
(21) Application number: 04020559.3
(22) Date of filing: 30.08.2004
(51) Int. Cl.: A61F 13/539

(54) **Disposable absorbent articles**

(30) Priority: 29.08.2003 US 652171
(71) Applicant: McNEIL-PPC, INC., Skillman, NJ 08858 (US)
(72) Inventor: Joseph, Annemarie Devine, Plainsboro, NJ 08536 (US); Lasko, Vincent P., New Egypt, NJ 08533 (US); O'Malley, Mary Gail, Brielle, NJ 08730 (US)
(74) Representative: Groening, Hans Wilhelm, Dipl.-Ing.

(57) **Abstract**

Absorbent articles incorporating an absorbent structure that includes an absorbent core having an upper surface and a lower surface, a tissue layer having an upper surface and a lower surface, and a self-cross-linking binder, wherein the lower surface of the first absorbent core is juxtaposed to the upper surface of the tissue and the binder applied to the lower surface of the tissue layer such that the binder penetrates through the tissue to the absorbent core.

## Description

### BACKGROUND OF THE INVENTION

Disposable absorbent articles such as, pantiliners, sanitary napkins, interlabial devices, adult incontinence devices, bandages, and diapers are well known in the art. These articles typically have a fluid permeable body-facing side and fluid impermeable garment facing side and may include an absorbent core for retaining fluids therebetween. Such absorbent structures have traditionally been made from readily available and relatively inexpensive materials, such as, cotton fibers, wood pulp fluff, cellulosic tissue, or wadding, or other absorbent materials. These materials have provided satisfactory absorbency of fluids both in terms of absorbency rate and overall absorbent capacity.

It has been found that absorbent articles that are thin and flexible tend to offer enhanced fit and comfort. One way to obtain thinness is to decrease the amount of material in the absorbent core. Unfortunately, absorbency often decreases if the absorbent core is made from less material. Another way to obtain thinness is to compress the material. Unfortunately, the absorbent article may also become stiffer as the layers are compressed.

Another problem that absorbent structures may suffer from is the tendency to collapse ("wet collapse") when wetted, thereby losing some of their void volume. Such structures may also allow absorbed fluid to be squeezed back out of the structure onto the user of the absorbent article. Furthermore, when such structures have absorbed fluid, they may present an uncomfortable wet feeling against the skin of the user.

More recently, superabsorbent polymer particles have been combined with the more traditional absorbent materials to provide structures with enhanced absorbency and retention, which may help to eliminate the problems of squeeze-out and wet surface feel. Replacement of traditional absorbent materials with superabsorbent polymer particles may also allow for absorbent products to be thinner while retaining the absorbent capacity of thicker, bulkier products. A drawback to superabsorbent polymer particles, however, is their relatively high cost compared to the more traditional absorbent materials.

Additionally, since superabsorbent polymer particles tend to swell as they absorb fluid, they may cause what is commonly known as gel-blocking. In other words, as fluid is absorbed by the particles of superabsorbent polymer, those particles swell and may form an occlusive layer of swollen superabsorbent particles. This occlusive layer then prevents the passage of additional fluid into the structure. Thus, the superabsorbent polymer particles must be properly placed within an absorbent structure to allow for this swelling and to most fully utilize their absorbent capacity. Generally, prevention of gel-blocking has been realized by mixing superabsorbent polymer particles with spacer materials, such as, absorbent or nonabsorbent fibers, or by placing the superabsorbent polymer particles toward the bottom of the absorbent structure, e.g., away from the body. However, although these methods of superabsorbent polymer placement may minimize gel-blocking, they do not effect the most efficient use of the superabsorbent polymer's absorbent capacity.

Latex binders have also been used in a variety of nonwoven applications to enhance the absorbent structure of the resultant absorbent article. For example, binders are used to bind together loosely assembled masses of fibers to form a self-sustaining web, which can then be used to produce rolled goods that form an absorbent core.

Such binders may include one that incorporates cross-linking monomers into the latex. The cross-linking monomers may be chemically or thermally activated during processing of the web. Whatever the specific conditions, this cross-linking may help to improve the stability, absorption properties and/or durability of the nonwoven. See, for example, U.S. Pat. Pub. 2003/0039817 A1, U.S. Pat. Nos. 5,656,367; 6,433,245; and WO 02/060498 A2. Choosing the appropriate binder for a specific property may lead to other undesirable attributes. For example, a specific binder may enhance the stability of the absorbent material but may result in an absorbent structure that is rough to the touch. Another binder may improve the fluff of an absorbent material but the binder may require an acidic pH to be activated and thus requiring additional steps during processing.

Absorbent articles may include wicking layers in order to optimize fluid management and distribution. Wicking layers will typically have higher wicking capability than the absorbent material used in the absorbent structure and may be placed below the absorbent structure. For example, U.S, Pat. No. 5,454,800 discloses a tissue-wicking layer made from softwood and/or hardwood fibers, which can be creped, wet pressed or through-air dried. See also U.S. Pats. Nos. 5,873,869 and 6,492,574. Tissue may also be used to form the absorbent structure. For example, an absorbent article sold under the name of CAREFEE® Coverage Plus^{tm} Pantiliner (sold by Personal Products Company, Division of McNeil-PPC, Inc., Skillman, NJ) contains a tissue layer placed between the absorbent core and the barrier.

What has been surprisingly found is that when combining an absorbent core and a tissue layer to form an absorbent structure, one can add a self-crosslinking latex binder to achieve superior properties, such as, rapid fluid penetration, low rewet and drape stiffness that results in comfort and a desired thickness.

### SUMMARY OF THE INVENTION

The present invention is directed to an absorbent structure comprising, consisting of and/or consisting essentially of an absorbent core having an upper surface and a lower surface, a tissue layer having an upper surface and a lower surface, and a self-cross-linking binder, wherein the lower surface of the first absorbent core is juxtaposed to the upper surface of the tissue and the binder applied to the lower surface of the tissue layer such that the binder penetrates through the tissue to the absorbent core.

The present invention is also directed to an absorbent article comprising, consisting of and/or consisting essentially of an absorbent structure having an absorbent core having an upper surface and a lower surface, a tissue layer having an upper surface and a lower surface and a self-cross-linking binder, wherein the lower surface of the first absorbent core is juxtaposed to the upper surface of the tissue and the binder applied to the lower surface of the tissue layer such that the binder penetrates through the tissue to the absorbent core, and a barrier layer.

The present invention is further directed to an absorbent article comprising, consisting of and/or consisting essentially of a cover having a body contacting surface and an absorbent structure contacting surface, an absorbent structure comprising an absorbent core having an upper surface and a lower surface, a tissue layer having an upper surface and a lower surface and a self-cross-linking binder, wherein the lower surface of the first nonwoven material is juxtaposed to the upper surface of the tissue and the binder applied to the lower surface of the tissue layer such that the binder penetrates through the tissue to the absorbent core; and, a barrier layer.

### BRIEF DESCRIPTION OF THE DRAWINGS

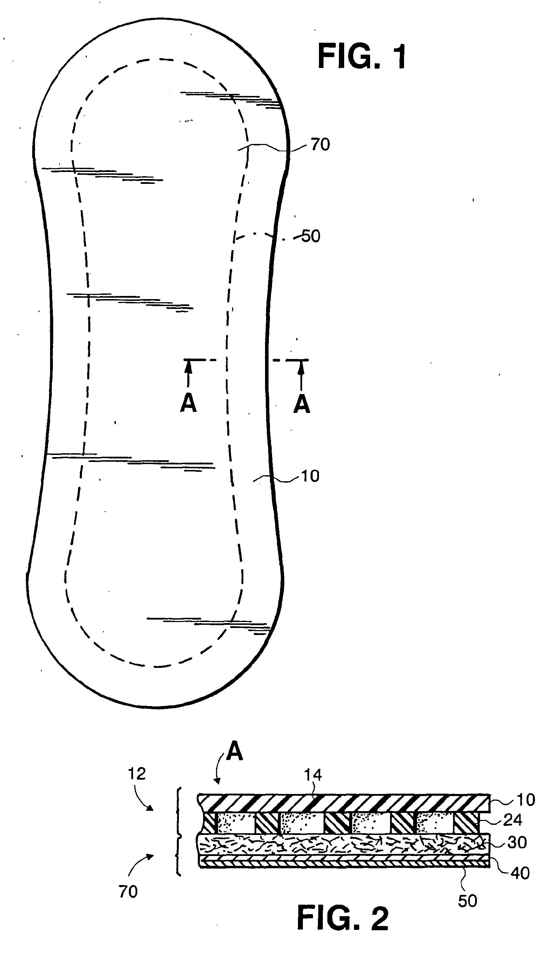

FIG. 1 is a top plan view of an absorbent article according to the invention and
FIG. 2 is a cross-sectional view taken across line A-A of the embodiment shown in FIG. 1.

### DETAILED DESCRIPTION OF THE INVENTION

This invention provides a disposable absorbent article including an absorbent structure containing an absorbent core, a tissue layer, and a self-cross-linking binder, where the binder is applied to the lower surface of the tissue layer such that the binder penetrates through the tissue to the nonwoven absorbent core. The absorbent structure provides the ability to control stiffness and increased wetability and wicking. Additionally, the invention also provides a cover-transfer layer laminate.

Disposable absorbent articles, such as, pantiliners, sanitary napkins, interlabial devices, adult incontinent devices, breast pads, shoe insoles, bandages, and diapers are well known in the art. These articles typically have a fluid permeable body-facing side and fluid impermeable garment facing side. Additionally, such articles may include an absorbent core for retaining fluids therebetween.

### Cover

Although not required, the absorbent article of the present invention may include a cover overlaying the absorbent material. The exterior of the cover would then form the body-facing surface of the absorbent article. As known by those skilled in the art, the cover may be formed from any fluid pervious material that is generally compliant, soft feeling, and non-irritating to the user's skin and permits fluid to penetrate to the absorbent core, which retains the fluid. The cover generally functions to transport fluid away from the wearer into the absorbent article. In this manner, fluid and moisture are removed from contacting the wearer, thus making the wearer feel dry and comfortable. In addition to transporting fluid, the cover may also absorb and/or retain fluid as well.

The cover can be made from any of the materials conventional for this type of use. Non-limiting examples of suitable materials that can be used as the cover layer 10 are woven and nonwoven fabrics formed from cellulose, polyester, polypropylene, nylon, and/or rayon fibers or the cover layer may be an apertured thermo-plastic film and formed films. Other materials used in making covers include gauze or any known porous material with a suitable body contacting surface, including, but not limited to nonwoven webs, plastic nets, and the like. The cover 10 could also be made from a fibrous nonwoven composite of bicomponent fibers and pulp fluff.

Bicomponent fibers are known in the art and are composed of two polymers with different melting points. At least a portion of the outer surface of each bicomponent fiber has the lower melting polymer. The two polymers may be arranged such that a cross-section of the fiber shows the two polymers in a side-by-side array. Alternatively, the polymers may be positioned in a so-called sheath/core arrangement, in which a core of higher melting polymer is surrounded by a sheath of lower melting polymer. A useful bicomponent fiber is a 3.0 denier, 1.5" long staple fiber made of a polyester core and a high density polyethylene sheath. Similar fibers (polyethylene sheath and polypropylene core) are available as Danaklon ES-C or ES Bico (Danaklon A/S, Varde Denmark). Pulp fibers may be obtained as IP "'SUPERSOFT" ELM supplied by the International Paper Company (Memphis, Tennessee), "'RAYFLOC" XJ-HM E-Type Cellulosic Fluff Pulp, (ITT Rayonier), or Korsnas Vigorfluf-EN White (KorsncAs, Gavle, Finland).

The cover layer 10 may optionally be treated with surfactant to manipulate the hydrophobicity/hydrophilicty thereof to facilitate optimal fluid transport properties. The fibers or other materials that make up the cover layer should not collapse or lose their resiliency when subjected to body fluid. The fibers may be oriented by a carding process and thermally bonded via embossing. The fiber or filament can be single denier or multidenier.

The thickness of the cover 10 may vary from about 0.025 mm to about 5 mm, depending on the material chosen. The weight of the body-facing layer material should be between about 5 to about 150 grams per square meter (gsm).

Generally, the optional cover is a single sheet of material having a width sufficient to form the body-facing surface of the absorbent article. The cover may be longer and wider than the absorbent core.

The cover 10 may be embossed with shapes within a given area. For example, a series or a number of features, e.g., circles, triangles, squares, lines, honeycomb, diamond, floral, etc. are embossed over the entire length and width of the outer surface of web. Each embossed feature has a major and minor axis extending therethrough, the major axis length being greater or equal to the minor axis length. The embossed features may be in a repetitive pattern.

In one embodiment of the invention, the cover layer 10 includes a spunlace nonwoven. In particular, the spunlace material may be made from about 0 to about 100% rayon and from about 0 to about 100% polyester. The spunlace material may also be made from about 10 to about 65% rayon and from about 35 to about 90% polyester may be used. Optionally, the material used for the body-facing layer may include binders, such as, thermoplastic binder fibers and latex binders.

### Transfer layer

Optionally, the absorbent article of the present invention may include a transfer or distribution layer. The transfer layer or distribution layer, if present, is generally positioned beneath the cover 10 and the transfer layer usually directly contacts the absorbent core. If included, the transfer layer may be made of any known material that will take up fluid and then distribute and release it to an adjacent absorbent layer for storage. Transfer layers have a relatively open structure that allows for movement of fluid within the layer. Suitable materials for such transfer layers include fibrous webs, resilient foams, and the like.

The transfer layer provides a means of receiving body fluid from the fluid-pervious cover layer 10 and holding it until the absorbent core has an opportunity to absorb it. The transfer layer is, preferably, more dense than the cover layer 10 and has a larger proportion of smaller pores than does the cover layer 10. These attributes allow the transfer layer to contain body fluid and hold it away from the outer side of the cover layer 10, thereby preventing the fluid from re-wetting the cover layer 10 and its outer surface. However, the transfer layer is preferably not so dense as to prevent the passage of the fluid through the transfer layer and into the underlying absorbent core.

The transfer layer may include various materials, including, for example, fibrous webs, resilient foams, and the like. The transfer layer may include cellulose fibers such as from wood pulp, single component or bicomponent fibers that include thermoplastic materials (such as, polyester, polypropylene, polyethylene, among others) in fiber or other forms, rayon, organic binders (such as, copolymers of vinyl, acrylic and/or other monomers that may be coated onto thermoplastic fibers or otherwise incorporated into the transfer layer) among other materials known to the art. The transfer layer may, for example, have a basis weight in a range from about 40 gsm to about 120 gsm, a thickness in a range from about 0.5 mm to about 4 mm, a density in a range from about 0.03 g/cc to about 0.15 g/cc.

The mass of materials making up the transfer layer may be absorbent, although the materials themselves are not absorbent. Thus, transfer layers that are made of hydrophobic, nonabsorbent fibers may be able to accept large volumes of fluid into interfiber void spaces while the fibers themselves do not absorb any significant quantities of fluid. Likewise, open-celled foam structures that are made from nonabsorbent materials may also absorb fluid into the cells of the foam. The walls of the cells, however, do not absorb any fluid. The cumulative spaces within the transfer layer, i.e., the interfiber void spaces in the fibrous transfer layer or the open cells in the foam transfer layer, function much like a container to hold fluid.

Typically, transfer layer fibrous webs are made of resilient, nonabsorbent materials to provide void volume and to allow for free movement of fluid through the structure. Transfer layers that are made from webs of mostly absorbent fibers absorb the fluid as it enters the structure and do not distribute it throughout the rest of the structure as efficiently as webs containing non-absorbent materials.

Transfer layers that are made from webs of mostly absorbent fibers absorb the fluid as it enters the structure and do not distribute it throughout the rest of the structure as efficiently as webs containing non-absorbent materials. Preferred transfer-layer fibrous webs include nonabsorbent materials to provide void volume and to allow for free movement of fluid through the structure. Examples of such materials include polypropylene, polyethylene, polyester, bicomponent materials, nylon and mixtures or combinations thereof. The transfer layer does not have to be apertured film; it can be any other nonwoven material, such as, foam or netting, which transports fluid and in combination with the cover, provides masking of the absorbent core. However, in one embodiment, the transfer layer is a 25 gsm apertured film made from polyethylene.

### Cover/Transfer Layer Laminate

A laminate formed from the cover and transfer layers may be used. In an embodiment, the composite layer includes an embossed pattern on the outer surface. For example, flowers and rails depicted in U.S. Des. Pat. No. 439,057 are embossed after the composite is formed, which results in an embossed pattern having flowers, rails, and squares

In one embodiment of the present invention, the cover and transfer layers are joined to form a laminate 12. This two layer structure is particularly useful in personal care products such as feminine sanitary protection products having body-contacting, facing or cover layers, such as, transfer or fluid handling layers, or as other components of personal care products. The laminates of the invention have been found to exhibit improved fluid-handling properties when used in disposable absorbent articles, such as, for instance, feminine sanitary protection products.

The first layer 14, which is in one embodiment a body-contacting layer, may be made from any one of a variety of fluid permeable materials. As a body-contacting layer, the first layer 14 is preferably compliant, soft feeling, and non-irritating to a user's skin. The first layer 14 should further exhibit good strikethrough and a reduced tendency to rewet, permitting bodily discharges to rapidly penetrate it and flow toward subsequent underlying layers, while not allowing such discharges to flow back through the body-contacting layer to the skin of the user.

The first layer 14 may be made from a wide range of materials including, but not limited to, woven or knitted fabrics, nonwovens, apertured films, hydro-formed films, porous foams, reticulated foams, reticulated thermoplastic films, and thermoplastic scrims. In addition, the first layer 14 may be constructed from a combination of one or more of the above materials, such as, a composite layer of a nonwoven and apertured film.

Likewise, the second layer 24 may also be made from a variety of fluid permeable materials including, but not limited to woven or knitted fabrics, nonwovens, apertured films, hydro-formed films, porous foams, reticulated foams, reticulated thermoplastic films, thermoplastic scrims, and combinations thereof.

Nonwovens and apertured films are preferred for use as both the first layer 14 and the second layer 24. Suitable nonwovens may be made from any of a variety of fibers as known in the art. The fibers may vary in length from a quarter of an inch or less to an inch and a half or more. It is preferred that when using shorter fibers (including wood pulp fiber), the short fibers be blended with longer fibers. The fibers may be any of the well known artificial, natural or synthetic fibers, such as cotton, rayon, nylon, polyester, polyolefin, or the like. The nonwoven may be formed by any of the various techniques known in the art, such as, carding, air laying, wet laying, melt-blowing, spunbonding and the like.

Apertured films are typically made from a starting film that is a thin, continuous, uninterrupted film of thermoplastic polymeric material. This film may be vapor permeable or vapor impermeable; it may be embossed or unembossed; it may be corona-discharge treated on one or both of its major surfaces or it may be free of such corona-discharge treatment; it may be treated with a surface active agent after the film is formed by coating, spraying, or printing the surface active agent onto the film, or the surface active agent may be incorporated as a blend into the thermoplastic polymeric material before the film is formed. The film may comprise any thermoplastic polymeric material including, but not limited to, polyolefins, such as, high density polyethylene, linear low density polyethylene, low density polyethylene, polypropylene; copolymers of olefins and vinyl monomers, such as, copolymers of ethylene and vinyl acetate or vinyl chloride; polyamides; polyesters; polyvinyl alcohol and copolymers of olefins and acrylate monomers, such as, copolymers of ethylene and ethyl acrylate and ethylenemethacrylate. Films having mixtures of two or more of such polymeric materials may also be used. The machine direction (MD) and cross direction (CD) elongation of the starting film to be apertured should be at least 100% as determined according to ASTM Test No. D-882 as performed on an Instron test apparatus with a jaw speed of 50 inches/minute (127 cm/minute). The thickness of the starting film is preferably uniform and may range from about 0.5 to about 5 mils or about 0.0005 inch (0.0013 cm) to about 0.005 inch (0.076 cm). Coextruded films can be used, as can films that have been modified, e.g., by treatment with a surface-active agent. The starting film can be made by any known technique, such as casting, extrusion, or blowing.

Aperturing methods are known in the art. Typically, a starting film is placed onto the surface of a patterned support member. The film is subjected to a high fluid pressure differential while on the support member. The pressure differential of the fluid, which may be liquid or gaseous, causes the film to assume the surface pattern of the patterned support member. Portions of the film overlying apertures in the support member are ruptured by the fluid pressure differential to create an apertured film. A method of forming an apertured fibrous film is described in detail in commonly owned US Pat. No. 5,827,597, which is incorporated herein by reference.

In one embodiment, the first layer 14 and the second layer 24 contact one another substantially only through a plurality of spaced apart, disconnected macrofeatures. By this is meant the layers are joined to one another substantially only at macrofeatures. The macrofeatures may be located on the first layer or the second layer. When the macrofeatures are located on the first layer, they project in the direction of the second layer. When the macrofeatures are located on the second layer, they project in the direction of the first layer.

In another embodiment, the first layer 14 and the second layer 24 contact one another through macrofeatures and the surface of the film containing a plurality of apertures.

As used herein, the term "macrofeature" means a surface projection visible to the normal, unaided human eye at a perpendicular distance of about 300 mm between the eye and the surface. Preferably, the macrofeatures each have a maximum dimension of at least about 0.15 mm. More preferably, the macrofeatures each have a maximum dimension of at least about 0.305 mm. Most preferably, the macrofeatures each have a maximum dimension of at least about 0.50 mm. The macrofeatures are discrete and disconnected from one another. That is, if an imaginary plane, i.e., a first plane, were lowered onto the first surface of the three-dimensional layer, it would touch the layer at the top of the macrofeatures in multiple discrete areas separated from one another. It is not necessary for each and every macrofeature to touch the imaginary plane; rather, the first plane is thus defined by the uppermost portions of the macrofeatures, that is, those parts of the macrofeatures projecting the farthest from the second surface of the layer.

Where the layer with macrofeatures comprises an apertured film, the film has a first surface, a second surface, and a caliper defined by a first plane and a second plane. The film comprises a plurality of disconnected macrofeatures and a plurality of apertures. The apertures are defined by sidewalls that originate in the film's first surface and extend generally in the direction of the film's second surface to terminate in the second plane. The first surface of the film is coincident with the first plane at the disconnected macrofeatures.

Where the layer with macrofeatures comprises a nonwoven, the nonwoven has a first surface, a second surface, and a caliper defined by a first plane and a second plane. The nonwoven further comprises a plurality of disconnected macrofeatures, wherein the first surface of the nonwoven is coincident with the first plane at the disconnected macrofeatures.

In one embodiment, the macrofeatures are arranged in a regular pattern relative to each other. Moreover, if the macrofeatures project from a layer that is an apertured film, the macrofeatures and the apertures are arranged in a regular configuration relative to each other on said layer. The apertures and macrofeatures recur at fixed or uniform intervals with respect to one another. The spatial relationship between the apertures and the macrofeatures define a geometric pattern that is consistently repeated throughout the surface area of the film. The apertures and macrofeatures are arranged in a regular, defined pattern uniformly repeated throughout the film.

The apertures and macrofeatures may be arranged so that there are more apertures than macrofeatures, although the relative arrangement of apertures and macrofeatures is regular. The exact sizes and shapes of the apertures and macrofeatures are not critical, as long as the macrofeatures are large enough to be visible to a normal unaided human eye at a distance of about 300 mm, and as long as the macrofeatures are discrete and disconnected from one another.

In one embodiment, the first layer 14 and the second layer 24 contact one another substantially only though the macrofeatures. That is, the macrofeatures function much like spacers to hold the first layer 14 away from the surface of the second layer 24 except where they contact one another at the macrofeatures. Accordingly, fluid communication is provided around the macrofeatures. Fluid entering the space between the first layer and the second layer is directed around the macrofeatures. This advantageously distributes the fluid in the X-Y direction across the surface of the second layer. As a consequence, the fluid is also more readily transported downward through the structure in the Z direction since the X-Y spread provides more surface area through which the fluid can penetrate into the lower layers in the Z direction.

In another embodiment of the invention, the first layer 14 includes a nonwoven, while the second layer 24 includes either a nonwoven or an apertured film. The macrofeatures may be located on either the first layer or the second layer.

In yet another embodiment, the first layer 14 includes an apertured film, while the second layer 24 includes either a nonwoven or an apertured film. In this embodiment, the macrofeatures may also be located on either the first layer or the second layer. However, when the macrofeatures are present on the first layer, the macrofeatures on the first layer preferably contain apertures, i.e., apertured macrofeatures, and are disconnected from all other apertured macrofeatures on the first layer. Each apertured macrofeature is a discrete physical element.

Such a three-dimensional apertured film is preferably formed by placing a thermoplastic film across the surface of an apertured support member with a pattern of macrofeatures and apertures. A stream of hot air is directed against the film to raise its temperature to cause it to be softened. A vacuum is then applied to the film to cause it to conform to the shape of the surface of the support member. Portions of the film lying over the apertures in the support member are ruptured to create apertures in the film.

A suitable apertured support member for making these three-dimensional apertured films is a three-dimensional topographical support member made by laser sculpting a workpiece. The workpiece comprises a thin tubular cylinder. The workpiece has non-processed surface areas and a laser sculpted center portion. A preferred workpiece for producing the support member of this invention is a thin-walled seamless tube of acetal, which has been relieved of all residual internal stresses. The workpiece has a wall thickness of from about 1 to about 8 mm, more preferably from about 2.35 to about 6.5 mm. Exemplary workpieces for use in forming support members are one to six feet in diameter and have a length ranging from two to sixteen feet. However, these sizes are a matter of design choice. Other shapes and material compositions may be used for the workpiece, such as, acrylics, urethanes, polyesters, high molecular weight polyethylene and other polymers that can be processed by a laser beam.

Methods of making an apertured film are disclosed in copending application USSN# __/____, TWO LAYER STRUCTURE FOR ABSORBENT ARTICLES, filed 08/29/2003 and is herein incorporated by reference in its entirety.

The two-layer structure may advantageously be used as a cover/transfer layer of an absorbent article, such as, a sanitary napkin, pantiliner, diaper, incontinence pad, or other similar product for absorbing exudates from the body, such as, menses, urine, feces, or sweat.

### Absorbent Structure

The absorbent structure 70 of the present invention may contain an absorbent core 30 made from any known absorbent materials including, but not limited to, absorbent fibers, such as, cellulose fibers, including, but not limited to wood pulp, regenerated cellulose fibers, and cotton fibers, rayon fibers and the like; superabsorbent powders (SAP) like Sumitomo SA-70 or fibers (SAF), other naturally occurring absorbent materials, such as, sphagnum or peat moss; and other synthetic absorbent materials, such as, foams and the like. The absorbent core 30 may also include one or more of the following: binders, such as, thermoplastic and latex, odor-controlling compounds, e.g., perfumes, EDTA (ethylenediaminetetraacetic acid), anti-microbial agents, wetting agents, wetness indicator material, materials for administering or delivering medicaments, such as encapsulated medicaments, and materials for maintaining skin moisture, such as encapsulated moisturizers.

For example, the absorbent core 30 may be made from material such as a fluffy batt cut from a relatively loose web of non-woven fibers having a relatively high absorptive capacity. While the absorbent core can have any shape or silhouette, it usually has an asymmetric configuration. The absorbent core 30 may also be made from material such as a fibrous batt having an integral densified layer. In such a case, if a backsheet is desired, the absorbent core is positioned on the backsheet of the absorbent article so that the densified layer adjoins the backsheet. The densified layer has relatively higher wettability and liquid retentivity than the rest of the aforesaid batt and usually is formed by slightly moistening one surface of the batt and thereafter compressing the moistened surface. The absorbent core 30 may also be formed from multiple layers, each having a different density such that the uppermost layer (closest to the body) is less dense than the outer (closest to the garment).

Additionally, the absorbent core 30 may be formed of absorbent material made from an offline-formed, homogeneously mixed, air-laid layer, roll good laminate or any other offline-formed absorbent composite.

The absorbent material of this invention may be made from a number of processes, including, but not limited to, airlaying, spunbonding, bonding and carding, meltblowing, and coforming. In one embodiment, the absorbent core is absorbent core air-laid pulp.

The air laid process is well known. A fibrous nonwoven composite made from Buckeye Foley Fluff^{TM} (Memphis, TN) pulp, Trevira or KoSa T255 (Houston, TX) bicomponent fibers can be formed by the separation of bundles of short fibers entrained into an air stream. These fibers are deposited onto a forming screen, typically a horizontal or rotary drum, with the aid of a vacuum supply. There may be multiple forming sections (forming heads). The random web is bonded together via a hot-air activated latex-bonding adhesive, thermal bonding fibers, or combination as taught in, for example, U.S. Pat. Nos. 4,640,810 and 5,885,516.

The binder may be in the form of fibers, liquid or particles. Binders may aid in preventing wet collapse of the material. Suitable fiber binders that can be used with this invention include sheath core conjugate fibers available from KoSa Inc. (Houston, TX) under the designation T-255 and T-256, both with a polyolefin sheath, or T-254, which has a low-melt co-polyester sheath. Other fiber binders are known to those skilled in the art, and are available by many manufacturers such as Chisso and Fibervisions LLC of Wilmington, Del. A suitable liquid binder is KYMENE® 557LX available from Hercules, Inc. of Wilmington, Del. Other suitable liquid binders include ethylene vinyl acetate emulsion polymers sold by National Starch and Chemical Company (Bridgewater, N.J.) under the tradename DUR-O-SET® ELITE ® series (including ELITE® 33 and ELITE® 22). Other suitable binders are sold by Air Products Polymers and Chemicals under the name AIRFLEX®.

Some binders require catalysts, elevated temperatures, and/or acidic conditions in order to cross-link. Monomers derived from the reaction of a polymerizable amide with an aldehyde are conventionally used for such purposes. The binder can be applied to the top, bottom, or both of the layers at various add on levels. Different types of latex binders, e.g., high Tg, e.g., about +10 to about +35°C, or low Tg, e.g, about -3 to about -30°C, those binders having Tg between the high and low have differing degrees of stiffness and softness and may be applied independently or in combination depending on the desired properties of the resulting structure. See for example, U.S. Pat. Nos. 4,449,978 and 5,143,954. Other binders such as those disclosed in U.S. Pat. No. 5,415,926, the entire disclosure incorporated herein in its entirety, are considered to be self-crosslinking. In these polymer systems, a reactive functional group allows the polymer to crosslink with itself, as well as chemically bond to the substrate such as pulp or tissue. The self-crosslinking reaction can be accelerated through the use of acid catalysts. An example of a self-crosslinking binder includes "X-LINK 25-033A", a self-crosslinking vinyl acrylic copolymer emulsion having a high glass transition temperature, available from National Starch and Chemical Company (Bridgewater, NJ).

The absorbent material may also be deposited onto a carrier substrate, e.g., tissue 40 or other air permeable composition to form an absorbent structure 70. In this configuration, the layers are typically sprayed with a binder to stabilize the resulting absorbent structure 70. The structure 70 can further be pattern embossed to achieve aesthetics and/or functionality, e.g., wicking, densification, and the like. The resulting structure can be used as an absorbent structure into absorbent products, such as, sanitary napkins or pantiliners.

In one embodiment, the absorbent structure 70 contains an absorbent core 30 made from absorbent material and a tissue layer 40. In one embodiment, the tissue layer 40 is placed between the absorbent material and barrier layer 50. The tissue layer may be made from softwood and/or hardwood fibers and can be creped, wet pressed, or through-air dried.

Other additives may be incorporated into the absorbent core, such as, surfactants, SAP, and SAF. These additives may provide additional benefits such as enhanced fluid penetration and increased fluid absorption. For example, in one embodiment, the absorbent layer is made of absorbent material that is made from a layer of pulp. In another embodiment, SAP is mixed with the pulp to form an absorbent composite. This composite may be condensed to form a dense, thin layer. One example of such a material is Novathin® available from Rayonier, Jesup, GA.

SAP are particles that are capable of absorbing many times, at least 10, more preferably 15, and still more preferably over 15, their weight in exudate, under a pressure of 0.5 psi. It should be noted that, in the context of the present invention, there is no restriction that the superabsorbent particles actually be particulate. This expression is intended to cover superabsorbent fibers, and other superabsorbent materials, whatever their form and shape. These superabsorbent particles generally fall into three classes, namely starch graft copolymers, cross-linked carboxymethylcellulose derivates and modified hydrophilic polyacrylates. Examples of such absorbent polymers are hydrolyzed starch-acrylonitrile copolymer graft copolymer, a neutralized starch-acrylic acid graft copolymer, a saponified acrylic acid ester-vinyl acetate copolymer, a hydrolyzed acrylonitrile copolymer or acrylamide copolymer, a modified cross-linked polyvinyl alcohol, a neutralized self-cross-linking polyacrylic acid, a cross-linked polyacrylate salt, carboxylated cellulose, and a neutralized cross-linked isobutylene-malasic anhydride copolymer. In one embodiment of the invention, the superabsorbent particle is a cross-linked polyacrylate salt.

The superabsorbent particles are incorporated into the absorbent core in an amount no greater than about 60% on a weight per weight basis. Preferably, they are incorporated in an amount between about 0% and about 25% on a weight per weight basis. More preferably, they are incorporated in an amount between about 5% and about 20% on a weight per weight basis. For example, in the present context, 7% superabsorbent on "a weight per weight basis" is meant to mean 0.07 grams of superabsorbent particles per 1 gram of all components in the absorbent core.

The absorbent layer or core of the present invention may be constructed according to conventional techniques, e.g., by air-laying a mixture of wood pulp fibers and superabsorbent material. All such conventional techniques are within the scope of the present invention. In one embodiment, an absorbent layer is as described in U.S. Pat. No. 5,866,242, which is herein incorporated by reference in its entirety.

The ratio of SAP to wood pulp may be varied over a wide range. If desired, a layer or multilayer of drylaid type material can be used as the absorbent material to form the absorbent core. The absorbent material may be made of a SAP of the type used in the art and wood pulp fibers having the desired density.

Any tissue known in the art may be used to produce an absorbent structure 70 of the present invention, e.g., air-laid tissue and a wet-laid tissue.

### Barrier Layer

The barrier layer, also called backsheet 50, may be located adjacent to the absorbent structure 70 and to the cover 10 in portions elsewhere. The barrier layer 50 of the present invention is a body fluid impervious material, which is at least substantially impermeable to liquids. Its exterior forms the garment-facing surface of the absorbent article. The backsheet 50 may be any thin, flexible, body-fluid impermeable material, such as, but not limited to, a polymeric film, e.g., polyethylene, polypropylene, or cellophane, or a normally fluid pervious material that has been treated to be impervious, such as impregnated fluid repellent paper or non-woven material, including nonwoven fabric material, or a flexible foam, such as polyurethane or cross-linked polyethylene.

Optionally, the backsheet 50 may be breathable, i.e., permits vapor to transpire. Known materials for this purpose include nonwoven materials, monolithic and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet.

### Bonding Methods

The layers of the absorbent article may be, but not necessarily, bonded, e.g., glued or adhered, to the adjacent layer. For example, the underside of the cover 10 may be adhered to the topside of the absorbent structure 70. The underside of the absorbent structure 70 may be adhered to the topside of the barrier layer 50. Any methods known in the art, such as, fusion bonding, adhesive attachment, or by any other securement means can be used to secure the individual layers together to form the final absorbent article. Included within such methods are coembossing, thermobonding, mechanical bonding, and the like. Fusion bonding includes heat bonding, ultrasonic bonding, and the like.

Adhesive is typically used to attach the layers into a single absorbent article. For example, in one embodiment, the body facing cover 10 is attached to the barrier layer 50 with adhesive HL 1491 available from H.B Fuller and Company (St. Paul, MN). The adhesive may be applied in any method.

Adhesive may include pressure sensitive adhesive that is applied as strips, swirls, or waves, and the like. As used herein, the term pressure-sensitive adhesive refers to any releasable adhesive or releasable tenacious means. Suitable adhesive compositions, include, for example, water-based pressure-sensitive adhesives such as acrylate adhesives. Alternatively, the adhesive composition may include adhesives based on the following: emulsion or solvent-borne adhesives of natural or synthetic polyisoprene, styrenebutadiene, or polyacrylate, vinyl acetate copolymer or combinations thereof; hot melt adhesives based on suitable block copoylmers - suitable block copolymers for use in the invention include linear or radial co-polymer structures having the formula (A-B)x wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalphamethylstyrene, Polyvinyltoluene, and combinations thereof. Suitable Block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof. Commercial examples of these types of block copolymers include Kraton^{TM} elastomers from Shell Chemical Company, Vector^{TM} elastomers from Dexco, SolpreneTM from Enichem Elastomers and Stereon™ from Firestone Tire & Rubber Co.; hot melt adhesive based on olefin polymers and copolymers where in the olefin polymer is a terpolymer of ethylene and a comonomers, such as vinyl acetate, acrylic acid, methacrylic acid, ethyl acrylate, methyl acrylate, n-butyl acrylate vinyl silane or maleic anhydride. Commercial examples of these types of polymers include Ateva( polymers from AT plastics), Nucrel( polymers from DuPont), Escor (from Exxon Chemical).

The absorbent article of the present invention may be applied to the crotch of a garment by placing the garment-facing surface against the inside surface of the crotch of the garment. Various methods of attaching absorbent articles may be used. For example, chemical means, e.g., adhesive, and mechanical attachment means, e.g., clips, laces, ties, and interlocking devices, e.g., snaps, buttons, VELCRO (Velcro USA, Inc., Manchester, NH), zipper, and the like are examples of the various options available to the artisan.

Adhesive may be applied to the garment-facing side of the absorbent article. The positioning adhesive may be any adhesive known in the art. As a non-limiting example, pressure sensitive adhesive strips, swirls, or waves may be applied to help maintain the absorbent article in place. As used herein, the term pressure-sensitive adhesive refers to any releasable adhesive, or releasable tenacious means. Suitable adhesive compositions, include, for example, water-based pressure-sensitive adhesives, such as acrylate adhesives. Alternatively, the adhesive composition may include rapid setting thermoplastic "hot melt," rubber adhesives, two-sided adhesive tape, and the like.

Where positioning adhesive is used on the garment-facing side of the barrier layer 50, a release strip may be applied to protect the adhesive on the absorbent article prior to attaching the absorbent article to the crotch. The release strip can be formed from any suitable sheet-like material that adheres with sufficient tenacity to the adhesive to remain in place prior to use but which can be readily removed when the absorbent article is to be used. Optionally, a coating may be applied to release strip to improve the ease of removabilty of the release strip from the adhesive. Any coating capable of achieving this result may be used, e.g., silicone.

### Wings

Wings, also called, among other things, flaps or tabs, may also be part of the absorbent article of the present invention. Wings and their use in sanitary protection articles are described in U.S. Patent. No. 4,687,478 to Van Tilburg; U.S. Patent No. 4,589,876 also to Van Tilburg, U.S. Patent No. 4,900,320 to McCoy, and U.S. Patent No. 4,608,047 to Mattingly. The disclosures of these patents are incorporated herein by reference in their entirety.

As disclosed in the above documents, wings are, generally speaking, flexible and configured to be folded over the edges of the underwear so that the wings are disposed between the edges of the underwear.

### Miscellaneous

Any or all of the cover, absorbent layer, transfer layer, backsheet layer, and adhesive layers may be colored. Such coloring includes, but is not limited to, white, black, red, yellow, blue, orange, green, violet, and mixtures thereof. Color may be imparted according the present invention through dying, pigmentation, and printing. Colorants used according the present invention include dyes and inorganic and organic pigments. The dyes include, but are not limited to, anthraquinone dyes (Solvent Red 111, Disperse Violet 1, Solvent Blue 56, and Solvent Green 3), Xanthene dyes (Solvent Green 4, Acid Red 52, Basic Red 1, and Solvent Orange 63), azine dyes (Jet black), and the like.

Inorganic pigments include, but are not limited to, titanium dioxide (white), carbon black (black), iron oxides (red, yellow, and brown), chromium oxide (green), ferric ammonium ferrocyanide (blue), and the like.

Organic pigments include, but are not limited to diarylide yellow AAOA (Pigment Yellow 12), diarylide yellow AAOT (Pigment Yellow 14), phthalocyanine blue (Pigment Blue 15), lithol red (Pigment Red 49:1), Red Lake C (Pigment Red), and the like.

The absorbent article may be packaged as unwrapped absorbent articles within a carton, box or bag. The consumer withdraws the ready-to-use article as needed. The absorbent article may also be individually packaged (each absorbent article encased within an overwrap).

Also contemplated herein include asymmetrical and symmetrical articles having parallel longitudinal edges, dog bone- or peanut-shaped, circular, oval and the like.

An absorbent article of the present invention may be used with conventional underwear or may be shaped to conform to thong garments. As used herein, the term thong includes, but is not limited to, thong underwear, thong swimming suit bottom, G-strings, Rio cut underwear, Rio cut swimming suit bottom, Brazilian cut underwear, Brazilian cut swimming suit bottom, and any other garment that exposes the buttocks, having a narrow strip of fabric or a cord that passes between the thighs supported by a waistband, a waist cord, belt or the garment itself. The absorbent article may include other known materials, layers, and additives, such as, foam, net-like material, perfumes, medicaments or pharmaceutical agents, moisturizers, odor control agents, and the like. The absorbent article can optionally be embossed with decorative designs.

From the foregoing description, one skilled in the art can ascertain the essential characteristics of this invention, and without departing from the spirit and scope thereof, can make various changes and modifications. Embodiments set forth by way of illustration are not intended as limitations on the variations possible in practicing the present invention.

### EXAMPLES

### Example 1.

A layered composite made from Buckeye Foley Fluff™ (Memphis, TN) pulp, Trevira or KoSa T255 (Houston, TX ) bicomponent fibers, and 10% superabsorbent powders were deposited on a Cellu Type 3024 (Cellu Tissue Holdings (East Hartford, CT) tissue carrier. The binder was sprayed on the pulp side and the tissue side of the absorbent structure using a Nordson spray-type nozzle. The binder used was a self-cross-linking vinyl acrylic copolymer emulsion, disclosed in U.S. Pat. No. 5,415,926, available from National Starch & Chemical, Bridgewater, NJ, as X-LINK 25-033A. The binder was added to the tissue side at a load of 5% by wt and to the pulp side at a load of 3% by wt to give a total load of about 8% by weight resulting absorbent structure of binder.

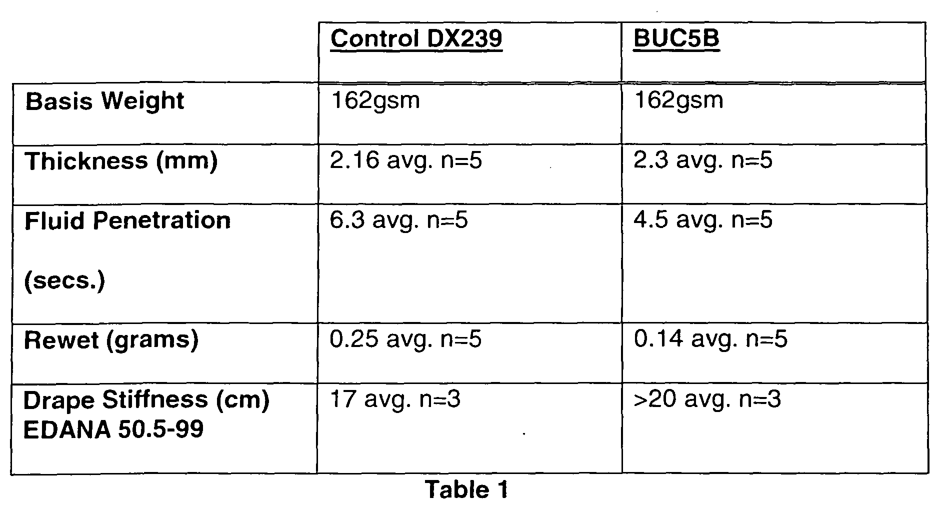

As the table above indicates, the BUC5B sample exhibited several advantageous properties over the control sample, including, increased bulk at the same basis weight, increased fluid penetration time, lower fluid rewet values, and increased stiffness. The overall product would have increased bulk and better fluid management. The increased stiffness aids in the antibunching attribute of the product.

Thickness measurements were made using an Ames Digital Thickness gauge. The gauge has a foot of 2.86 cm or 1.125 inches in diameter and also a dead weight of 56.7 g or 2 ounces. This results in a thickness measured under a pressure of .12 psig. The foot was raised and a sample was centered underneath. The foot is slowly lowered to rest on the sample. Readout was stabilized for approximately 5 seconds before reading was recorded.

Penetration times and rewets were determined by using a ½ inch plexiglass orifice plate with ½ inch diameter opening for dispensing the test fluid and a disposable syringe capable of dispensing 1 cc of artificial synthetic menstrual fluid testing fluid. The plexiglass orifice was placed onto the center portion of the samples. With a digital stopwatch accurate to .01 seconds, the time required when the test fluid touches the surface of the sample and until last of the fluid is absorbed was recorded.

For determining the rewet or wet back, a series of 15 ply Whatman # 1 filter papers were weighed and the weight accurate to about 0.01 grams was recorded. A layered composite was made and stained as in the above penetration test procedure. The single weighed filter paper was placed on top of the stain area and then a support-plate plexiglass, 10x3x.2", was placed on top of the filter paper. A 2 kilogram weight was placed on top of support plate for 15 seconds. After 15 seconds, the weight, support plate and filter paper were removed. The filter paper with fluid on it was reweighed and recorded. The dry filter paper weight was subtracted from the wet filter paper weight. This value is the Rewet or Wet Back value.

Drape values were determined by using a one-inch precision cut rectangular strip that was supported on a horizontal platform with the long axis of strip parallel to the long axis of the platform. The strip was advanced in the direction of its length so that an increasing part overhangs and bends down under its own weight. The overhang was free on one end and fixed at the other due to the pressure applied by the slide on the part of strip that was still on the platform. When the leading edge of the strip has reached the incline at an angle of 41.5 degrees below the horizontal, the reading (in cm) was recorded. This procedure was repeated on other side of material and average of both readings to obtain the final value.

### Example 2.

An additional sample was produced in the same manner as described in Example 1, except this sample had a different binder sprayed on the pulp side of the absorbent structure. This binder was a non-cross-linking acrylic copolymer emulsion binder available from National Starch and Chemical NACRYLIC™ 4401. The binder sprayed on the tissue side of the absorbent structure was the same as used in Example 1. The amounts of binder sprayed on the pulp and the tissue side of the absorbent structure were identical. The addition of the 4401 binder provides softness, drape and heat-sealability to the absorbent structure. The resulting sample had equivalent properties as in Example 1, but also had a softer feel on the top of the absorbent core. Additionally, the heat-sealability of the 4401 binder aids in the attachment of a cover to the absorbent structure and embossment of the finished product.

## Claims

1. An absorbent structure comprising:
a) an absorbent core having an upper surface and a lower surface,
b) a tissue layer having an upper surface and a lower surface, and
c) a self-cross-linking binder, wherein the lower surface of the first absorbent core is juxtaposed to the upper surface of the tissue and the binder applied to the lower surface of the tissue layer such that the binder penetrates through the tissue to the absorbent core.

2. An absorbent structure of claim 1, wherein the self-cross-linking binder is a self-cross-linking vinyl acrylic copolymer.

3. An absorbent structure of claim 1, wherein the self-cross-linking binder has a glass transition temperature of at least about +10°C.

4. An absorbent article comprising an absorbent structure of claim 1.

5. An absorbent structure of claim 1, wherein the self-cross-linking binder is applied to the upper surface of the absorbent core.

6. An absorbent structure of claim 1, further comprising a non-cross-linking binder wherein the non-cross-linking binder is applied to upper surface of the absorbent core.

7. An absorbent structure of claim 1, further comprising superabsorbent polymer.

8. An absorbent structure of claim 6, wherein the non-cross-linking binder has a glass transition temperature between the range of about -3 to about -30°C.

9. An absorbent structure of claim 8, wherein the self-cross-linking binder has a glass transition temperature of about +20 °C and the non-cross-linking binder has a glass transition temperature of about -23 °C.

10. An absorbent article comprising
a) an absorbent structure comprising an absorbent core having an upper surface and a lower surface, a tissue layer having an upper surface and a lower surface and a self-cross-linking binder, wherein the lower surface of the first absorbent core is juxtaposed to the upper surface of the tissue and the binder applied to the lower surface of the tissue layer such that the binder penetrates through the tissue to the absorbent core and
b) a barrier layer.

11. An absorbent article of claim 10, further comprising a non-cross-linking binder applied to the upper surface of the absorbent core.

12. An absorbent article of claim 10, further comprising a cover layer having an upper body-facing surface and a lower surface, the lower surface of the cover placed in juxtaposition to the upper surface of the absorbent core.

13. An absorbent article of claim 11 further comprising a cover layer having an upper body-facing surface and a lower surface, the lower surface of the cover placed in juxtaposition to the upper surface of the absorbent core.

14. An absorbent article of claim 10, wherein the cover layer comprises a laminate.

15. An absorbent article of claim 14, wherein the laminate comprises a nonwoven layer and an apertured film layer.

16. An absorbent article of claim 15, wherein the apertured film comprises raised macrofeatures.

17. An absorbent article of claim 16, where the nonwoven layer contacts the macrofeatures of the apertured film in the laminate.

18. An absorbent article of claim 10, wherein the self-cross-linking binder is a self-cross-linking vinyl acrylic copolymer.

19. An absorbent article of claim 10, wherein the self-cross-linking binder has a glass transition temperature of at least about +10°C.

20. An absorbent article of claim 11, wherein the self-cross-linking binder is about 5% by weight and the non-cross-linking binder is about 3% by weight of the absorbent structure.

21. An absorbent article of claim 10, wherein the self-cross-linking binder is applied to the upper surface of the absorbent core.

22. An absorbent article of claim 10, further comprising a non-cross-linking binder wherein the non-cross-linking binder is applied to upper surface of the absorbent core.

23. An absorbent article of claim 10, further comprising superabsorbent polymer.

24. An absorbent article of claim 22, wherein the non-cross-linking binder has a glass transition temperature between the range of about -3 to about -30°C.

25. An absorbent article of claim 22, wherein the self-cross-linking binder has a glass transition temperature of about +20 °C and the non-cross-linking binder has a glass transition temperature of about -23 °C.

26. An absorbent article comprising
a) a cover having a body contacting surface and an absorbent structure contacting surface;
b) an absorbent structure comprising an absorbent core having an upper surface and a lower surface, a tissue layer having an upper surface and a lower surface and a self -cross-linking binder, wherein the lower surface of the first nonwoven material is juxtaposed to the upper surface of the tissue and the binder applied to the lower surface of the tissue layer such that the binder penetrates through the tissue to the absorbent core; and
c) a barrier layer.

27. An absorbent article of claim 26, further comprising a non-cross-linking binder applied to the upper surface of the absorbent core.

28. An absorbent article of claim 26, further comprising superabsorbent polymer.

29. An absorbent article of claim 27, further comprising superabsorbent polymer.

30. An absorbent article of claim 26, wherein the cover layer comprises a laminate.

31. An absorbent article of claim 27, wherein the cover layer comprises a laminate.

32. An absorbent article of claim 28, wherein the cover layer comprises a laminate.

33. An absorbent article comprising
a) a cover having a body-contacting surface and an absorbent structure contacting surface;
b) an absorbent structure comprising a absorbent core having an upper surface and a lower surface, a tissue layer having an upper surface and a lower surface, a self-cross-linking binder and a non-cross-linking binder, wherein the lower surface of the first nonwoven material is juxtaposed to the upper surface of the tissue, the self-cross-linking binder applied to the lower surface of the tissue layer such that the binder penetrates through the tissue to the absorbent core and the non-cross-linking binder applied to the upper surface of the absorbent core; and
c) a barrier layer.
